## Europäisches Patentamt
## European Patent Office
## Office européen des brevets

(19)

(11) Veröffentlichungsnummer: **0 261 603 B1**

(12) **EUROPÄISCHE PATENTSCHRIFT**

(45) Veröffentlichungstag der Patentschrift: **08.05.91**

(51) Int. Cl.⁵: **C07D 307/36**

(21) Anmeldenummer: **87113687.5**

(22) Anmeldetag: **18.09.87**

(54) Verfahren zur Herstellung von Furan durch Decarbonylisierung von Furfural.

(30) Priorität: **23.09.86 DE 3632255**

(43) Veröffentlichungstag der Anmeldung:
**30.03.88 Patentblatt 88/13**

(45) Bekanntmachung des Hinweises auf die
Patenterteilung:
**08.05.91 Patentblatt 91/19**

(84) Benannte Vertragsstaaten:
**BE DE FR GB NL**

(56) Entgegenhaltungen:
**US-A- 3 223 714**
**US-A- 4 089 871**

**CHEMICAL ABSTRACTS, Band 77, Nr. 23, 4.
Dezember 1972, Seite 395, Zusammenfassung Nr. 151881d, Columbus, Ohio, US; &
SU-A-342 857 (S. HILLERS) 22-06-1972**

**CHEMICAL ABSTRACTS, Band 80, Nr. 9, 4.
März 1974, Seite 288, Zusammenfassung Nr.
47065s, Columbus, Ohio, US; O. RAUTAVUO-
MA: "Decarbonylation of furfural and benzaldehyde on a platinum catalyst", & KEM. TE-
OLLISUUS 1973, 30(10), 453-5**

**CHEMICAL ABSTRACTS, Band 86, Nr. 8, 21.**

**Februar 1977, Seite 513, Zusammenfassung
Nr. 43464x, Columbus, Ohio, US; G. GARDOS
et al.: "Investigation of furan production
from furfural over noble metal catalysts", &
HUNG. J. IND. CHEM. 1975, 3(4), 589-602**

(73) Patentinhaber: **BASF Aktiengesellschaft
Carl-Bosch-Strasse 38
W-6700 Ludwigshafen(DE)**

(72) Erfinder: **Wambach, Ludwig, Dr.
Humboldtstrasse 28
W-6900 Heidelberg(DE)**
Erfinder: **Irgang, Matthias, Dr.
Andreas-Hofer-Weg 41
W-6900 Heidelberg(DE)**
Erfinder: **Fischer, Martin, Dr.
Elbinger Weg 1
W-6700 Ludwigshafen(DE)**

## Beschreibung

Die vorliegende Erfindung betrifft ein Verfahren zur Herstellung von Furan durch Decarbonylierung von Furfural in der Gasphase in Gegenwart von Wasserstoff und einem Rhodium- oder Platin-Träger-Katalysator, der Alkali enthält.

Es ist bekannt, Furan durch Überleiten von gasförmigem Furfural mit Wasserdampf über Katalysatoren wie Kalk oder ein Gemisch von Zink- und Manganchromit herzustellen. Es sind auch Verfahren zur Decarbonylierung von Furfural in Gegenwart von Metallkatalysatoren, insbesondere Palladium, bekannt. Nach Literaturangaben besitzt Palladium die größte Aktivität, wohingegen Rhodium, Ruthenium, Platin und Nickel eine schwächere Aktivität haben sollen, z.B. bei Flüssigphasendecarbonylierung nach US-PS 3 007 941.

Die katalytische Decarbonylierung von Furfural ist in der Gas- oder in der Flüssigphase beschrieben. Für die Flüssigphasendecarbonylierung sind sowohl heterogene als auch homogene Katalysatoren bekannt. Für die heterogene Katalyse werden vor allem Edelmetallkatalysatoren, vorwiegend Palladiumkatalysatoren, verwendet. US-PS 3 007 941 beschreibt Palladiumkatalysatoren, die nach ihrer Produktivität bewertet werden. Unter Produktivität ist die Ausbeute an Furan pro Gramm Edelmetall während der gesamten Lebensdauer des Katalysators zu verstehen. Dabei ist die Produktivität vom beobachteten Zeitraum nach einer nicht offengelegten Rechenmethode bis zur Aktivität Null extrapoliert und es wird eine sogenannte "extrapolierte Katalysatorproduktivität zur Bewertung herangezogen. Die berechnete Produktivität liegt bei 21.000 g. Furan pro g Katalysator.

US-PS 3 257 417 beschreibt die Verwendung von Kaliumacetat als Base für die Aktivierung von Palladiumkatalysatoren für die Decarbonylierung von Furfural in der Flüssigphase. Bis auf 10 % des Anfangwertes der Aktivität werden 18 kg Furan pro g Palladium hergestellt.

Nach US-PS 3 223 714 verwendet man Palladiumkatalysatoren, welche mit Alkalibasen mit einem ph-Wert oberhalb von 11 aktiviert sind.

In SU-PS 342 857 wird die Herstellung von Silvan durch katalytische Decarbonylierung von Methylfurfural in Gegenwart von Katalysatoren, die Metalle der Gruppe VIII enthalten, aber besonders Palladium beschrieben. Die Katalysatoren werden mit Alkali promotiert. Extrapolierte Produktivitäten liegen nach 1.000 Stunden bei ungefähr 20 kg Methylfuran pro g Palladium, d.h. die Raumzeitausbeute ist unbefriedigend.

Für die technische Anwendung ist es erforderlich, Mindeststandzeiten für einen Katalysator von mehreren Wochen bis zur ersten Regenerierung bzw. bis zum Katalysatorwechsel bei entsprechend hoher Produktivität zu erreichen. Bei den bisher bekannten Verfahren ist Produktivität und Standzeitverhalten unbefriedigend.

Gegenstand der Erfindung ist ein Verfahren zur Herstellung von Furan durch Decarbonylierung von Furfural in der Gasphase bei Temperaturen von 250 bis 400°C und einem Druck von 0,1 bis 10 bar in Gegenwart von Wasserstoff und edelmetallhaltigen Katalysatoren, bei dem man platin- und/oder rhodiumhaltige Katalysatoren, die 0,1 bis 10,0 Gew.% Alkali enthalten, verwendet und bei der Reaktion ein Molverhältnis Wasserstoff/Furfural von mindestens 0,5 zu 1 einstellt.

Vorzugsweise verwendet man platin- und/oder rhodiumhaltige Katalysatoren, die 0,1 bis 10,0 Gew.% Caesium enthalten.

Überraschenderweise zeigen Katalysatoren mit Platin und Rhodium wesentlich bessere Produktivitäten und Standzeiten als palladiumhaltige. Ein Vergleich der Produktivitäten der erfindungsgemäßen Platin- und Rhodium-Katalysatoren mit den bekannten Palladium-Katalysatoren zeigt die starke Überlegenheit. Dies ist überraschend, weil bei anderen Verfahren, welche in der flüssigen Phase durchgeführt werden, Rhodium und Platin eine geringere Aktivität zugeschrieben wird (US-PS 3 007 941, EP-A-96913).

Die Decarbonylierung wird in der Gasphase in kontinuierlicher Arbeitsweise durchgeführt, dabei arbeitet man bei einer Temperatur von 250 bis 400°C, vorzugsweise bei 300 bis 350°C, und einem Druck von 0,1 bis 10 bar, vorzugsweise bei atmosphärischem Druck.

Das Verfahren kann sowohl im Rohrreaktor mit stückigem Katalysatormaterial als auch in der Wirbelschicht durchgeführt werden.

Als Katalysatoren verwendet man Platin- oder Rhodium-Trägerkatalysatoren, die Alkalimetalle oder Alkaliverbindungen enthalten. Der Edelmetallgehalt beträgt 0,01 bis 10 Gew.%, vorzugsweise 0,1 bis 2 Gew.%, jeweils bezogen auf die Gesamtmasse des Katalysators.

Der Gehalt an Alkali beträgt 0,1 bis 10,0 Gew.%. Zweckmäßig verwendet man Na-, K- und/oder bevorzugt Cs-Verbindungen. Als Alkaliverbindungen werden vorzugsweise die Salze von schwachen Säuren, d.h. von Säuren mit einer Dissoziationskonstante von weniger als $1.10^{-3}$ verwendet. Die Alkalimetalle liegen im fertigen Katalysator im allgemeinen in Form ihrer Oxide vor.

Die Katalysatoren werden im allgemeinen auf inerte und mechanisch feste Träger aufgetragen, z.B. sind

EP 0 261 603 B1

Aluminiumoxid. Titandioxid, Kieselsäure, Aluminiumsilikat, Zeolithe, Magnesiumsilikat oder Aktivkohle geeignet. Vorzugsweise wird Aluminiumoxid verwendet.

Die Katalysatorherstellung erfolgt in zwei Stufen. Zunächst kann man den Träger mit der erforderlichen Menge der Edelmetallsalz-Lösung, z.B. mit Nitratlösung, tränken, dann bei 70 bis 200 °C trocknen und bei 300 bis 700 °C tempern. In der zweiten Stufe kann man das Alkalimetall in Form seines Carbonates, Acetates, Hydroxids oder anderer löslicher Salze aufbringen. Nach dem zweiten Tränkvorgang wird der Katalysator im allgemeinen bei Temperaturen zwischen 70 und 200 °C, vorzugsweise 100 bis 140 °C, getrocknet. Der Katalysator kann anschließend bei Temperaturen zwischen 300 und 700 °C, vorzugsweise bei 500 bis 550 °C getempert werden.

Der so erhaltene Katalysator wird zweckmäßig vor Gebrauch mittels Wasserstoff reduziert. Dies kann bei Temperaturen zwischen 150 und 500 °C, vorzugsweise 250 bis 350 °C geschehen. Dabei kann der Wasserstoff in reiner Form oder in Verdünnung, z.B. mit Stickstoff, zugeführt werden. Die Dauer der Reduktion beträgt 2 Stunden bis 1 Tag, vorzugsweise 10 Stunden.

Die Decarbonylierung wird in Gegenwart von Wasserstoff als Trägergas durchgeführt, wobei der Wasserstoff auch die Aufgabe hat, die Standzeit des Katalysators zu verlängern. Das Wasserstoff/Furfural-Verhältnis kann in weiten Grenzen gewählt werden. Bevorzugt ist ein Molverhältnis Wasserstoff/Furfural zwischen 0,5 und 2 zu 1 und insbesondere 0,75 zu 1.

Vorteil der vorliegenden Erfindung ist es, bei hohem Durchsatz lange Standzeiten und entsprechend hohe Produktivitäten zu erhalten. So können, wie in den nachfolgenden Beispielen beschrieben, pro g Platin oder Rhodium 40 bis 80 kg Furan produziert werden, bis die Aktivität des Katalysators auf 70 % Umsatz absinkt, wobei der Katalysator mit 1,8 mol Furfural pro 100 g Katalysator und Stunde belastet wird. Im Beispiel 6 werden unter den gleichen Bedingungen sogar über 130 kg Furan produziert. Damit werden die dem Stand der Technik entsprechenden Produktivitäten um den Faktor 6 übertroffen.

3

Tabelle: Furanproduktivitäten der neu entwickelten Katalysatoren im Vergleich zum Stand der Technik

| Katalysator | Produktivität (g Furan/g Metall) | Temp. | Phase |
|---|---|---|---|
| Beispiel 1 (0,79 % Pt, 1,65 % Na$_2$O auf Al$_2$O$_3$) | 80.000 bis zur Restaktivität 70 % | 300–350°C | Gas |
| Beispiel 2 (0,75 % Pt, 2 % Cs$_2$CO$_3$ auf Al$_2$O$_3$) | 131.500 bis zur Restaktivität 70 % | 300–350°C | Gas |
| Beispiel 3 (1 % Rh, 1,5 % Na$_2$O auf Al$_2$O$_3$) | 40.000 bis zur Restaktivität 70 % | 300°C | Gas |
| 10 % Pd/C + Na$_2$CO$_3$ (US-PS 3 007 941) | 21.000 extrapoliert bis zur Restaktivität =0 % | 200°C | flüssig |
| 5 % Pd/Al$_2$O$_3$ + Ca(CH$_3$COO)$_2$ (US-PS 3 257 417) | 17.690 bis zur Restaktivität 10 % | 215°C | flüssig |
| 0,3 % Pd/Al$_2$O$_3$ + Na$_4$SiO$_4$ (US-PS 3 223 714) | 16.500 bis zur Restaktivität 10 % | 300°C | Gas |
| 2 % Pd/C + 2 % Cs$_2$CO$_3$ (SU-PS 3 342 857) | 20.000 g Silvan bis nur noch 80 % der Anfangsaktivität | 240–320°C | Gas |

Katalysator A

600 g Al$_2$O$_3$-Strangpreßlinge werden mit 360 ml Platinnitrat-Lösung (1,5 % Pt) imprägniert. Die Trocknung erfolgt bei 120°C, die Temperung bei 520°C. Anschließend wird mit einer Lösung von 15 g Na$_2$CO$_3$ in 360 ml Wasser getränkt und wiederum bei 120°C getrocknet und bei 520°C getempert. Der fertige Katalysator enthält 1,65 Gew.% Na$_2$O und 0,79 % Pt.

4

Katalysator B

Man verfährt wie bei Katalysator A, setzt jedoch für die zweite Tränkung eine Lösung von 27 g $Cs_2CO_3$ in 360 ml Wasser ein. Der Katalysator enthält 0,75 % Pt und 4 % $Cs_2CO_3$.

Katalysator C

650 Strangpreßlinge aus Gamma-$Al_2O_3$ werden mit 365 ml einer Rhodiumnitratlösung (1,8 % Rh) getränkt. Das erhaltene Produkt wird bei 120° C getrocknet, bei 520° C getempert und nochmals mit einer Lösung von 17 g $Na_2CO_3$ in 345 ml Wasser getränkt. Der Katalysator wird wiederum bei 120° C getrocknet, bei 520° C getempert und enthält dann 1,0 Gew.% Rh und 1,5 Gew.% $Na_2O$.

Beispiel 1

Die Umsetzung erfolgt in einem senkrecht stehenden, elektrisch beheizten Rohrreaktor mit einem inneren Durchmesser von 35 mm und einer Höhe von 450 mm. In das Reaktionsrohr füllt man 100 g Trägerkatalysator A ein und erhitzt auf 300° C. Über diesen Katalysator führt man kontinuierlich ein Gasgemisch aus Furfural und Wasserstoff im Gleichstrom. Die Einspeisegeschwindigkeit beträgt 1,8 mol Furfural pro Stunde und 1,34 mol Wasserstoff pro Stunde. Aus dem Reaktionsgemisch wird Furfural ausgeschieden und rückgeführt, während man Furan in Kühlfallen kondensiert und gaschromatographisch analysiert. Als Nebenprodukt findet man wenig Methylfuran neben Spuren von n-Butanal und Propen.

Der anfängliche Umsatz beträgt 93 %, fällt dann aber laufend ab. Nach 510 Stunden kontinuierlichen Betriebs wird die Reaktionstemperatur auf 350° C angehoben, da der Umsatz auf 70 % gesunken ist. Die Temperaturerhöhung bewirkt wieder einen höheren Umsatz, der aber nach 590 Stunden erneut auf 70 % abfällt. Daher wird nach 590 Stunden abgestellt. Bis dahin werden pro g Platin 80 kg Furan mit einer durchschnittlichen Selektivität von 90 % hergestellt.

Beispiel 2

Man verfährt wie in Beispiel 1 und setzt einen Rhodiumkatalysator C mit 1 % Rh, 1,5 % $Na_2O$ auf $Al_2O_3$ auf $Al_2O_3$ ein. Die Selektivität schwankt zwischen 91 % und 95 % bei einem Umsatz von 90 % fallend auf 70 % nach 390 Stunden. In dieser Zeit werden 40 kg Furan pro g Rhodium hergestellt.

Beispiel 3

In den in Beispiel 1 beschriebenen Rohrreaktor füllt man 100 g Katalysator B ein. Man verfährt wie in Beispiel 1, erhöht aber nach den ersten 300 Betriebsstunden die Reaktortemperatur von 300 auf 310° C. Alle weiteren 200 Betriebsstunden erhöht man nun um weitere 10° C. Die Selektivität liegt anfangs bei 90 % und steigt kontinuierlich auf 95 % nach 1430 Stunden. Der Umsatz beträgt anfangs 99 %. Nach 1430 Betriebsstunden werden noch 70 % Umsatz erreicht. In dieser Zeit werden 131,5 kg Furan produziert.

**Ansprüche**

1. Verfahren zur Herstellung von Furan durch Decarbonylierung von Furfural in der Gasphase bei Temperaturen von 250 bis 400° C und einem Druck von 0,1 bis 10 bar in Gegenwart von Wasserstoff und edelmetallhaltigen Katalysatoren, dadurch gekennzeichnet, daß man platin-und/oder rhodiumhaltige Katalysatoren, die 0,1 bis 10,0 Gew.% Alkali enthalten, verwendet und bei der Reaktion ein Molverhältnis Wasserstoff/Furfural von mindestens 0,5 zu 1 einstellt.

2. Verfahren nach Anspruch 1, dadurch gekennzeichnet, daß man platin-und/oder rhodiumhaltige Katalysatoren verwendet, die 0,1 bis 10,0 Gew.% Caesium enthalten.

## Claims

1. A process for the preparation of furan by decarbonylation of furfural in the gas phase at from 250 to 400°C under from 0.1 to 10 bar in the presence of hydrogen and a catalyst containing noble metals, wherein a catalyst which contains platinum and/or rhodium and contains from 0.1 to 10.0% by weight of an alkali metal is used and the molar ratio of hydrogen to furfural is brought to not less than 0.5:1 in the reaction.

2. A process as claimed in claim 1, wherein a catalyst which contains platinum and/or rhodium and contains from 0.1 to 10.0% by weight of cesium is used.

## Revendications

1. Procédé de préparation de furanne par décarbonylation de furfural en phase gazeuse à des températures de 250 à 400°C et sous une pression de 0,1 à 10 bars en présence d'hydrogène et de catalyseurs contenant des métaux précieux, caractérisé en ce qu'on utilise des catalyseurs contenant du platine et/ou du rhodium qui contiennent de 0,1 à 10,0% en poids d'un métal alcalin et on règle, au cours de la réaction, un rapport molaire hydrogène/furfural d'au moins 0,5 à 1.

2. Procédé selon la revendication 1, caractérisé en ce qu'on utilise des catalyseurs contenant du platine et/ou du rhodium qui contiennent de 0,1 à 10,0% en poids de césium.